# EUROPEAN PATENT APPLICATION

(11) **EP 2 037 268 A1**
(43) Date of publication of application: **18.03.2009**
(21) Application number: 07116414.9
(22) Date of filing: 14.09.2007
(51) Int. Cl.: G01N 33/487

(54) **Apparatus and method for recording electrical activity in cells**

(71) Applicant: Aleria Biodevices, s.l., 08028 Barcelona (ES)
(72) Inventor: Claverol Tinturé, Enric Aleria Biodevices, S. L., 08028 Barcelona (ES)
(74) Representative: Barlocci, Anna

(57) **Abstract**

Apparatus for recording electrical activity produced by cells in-vitro, which comprises a device having a polymeric structure (12) comprising a well (14) for housing and culturing cells, a duct (15) disposed at right angles to said well and connected to its bottom region, and two electrodes (13,13') for recording a potential in said well, wherein the width of said well is at least ten times, preferably twenty times and more preferably fifty times, the width of said duct, and the width of at least of said electrodes (13) is at least ten times the width of said duct. The polymeric structure preferably comprises two such wells (14,14') connected by said duct (15), so that one of the electrodes can be placed in one well and another one of the electrodes can be placed in the other well.

## Description

The present invention relates to a device for recording electrical activity produced by cells in-vitro, comprising a polymeric structure which comprises a well or chamber for housing and culturing cells, a duct connected to the bottom region of said well, and two electrodes for recording a potential in said well, and relates to a method of manufacturing such a device too. The invention also relates to an apparatus and method for recording cellular electrical activity in-vitro.

### BACKGROUND ART

Electrogenic cells are cells that can alter their membrane potentials. Examples of electrogenic cells are nerve cells and myocytes. Neurons, for example, are capable of transiently modifying their membrane potential to sense, transport and modify information in the nervous system, and to control the contraction of muscles to perform motor actions. A large number of pathologies affecting the nervous system originate as a result of aberrant electrical activity in subpopulations of neurons. A common approach to find effective treatments relies on the identification of compounds which produce a controlled correction in the pattern of activity in a target cell population and a beneficial change in the symptoms displayed by the patient.

Electrophysiology is the study of the electrical properties of biological cells and tissues. It specifically includes measurements of the electrical activity of neurons, and particularly action potential activity, that is, the generation of a spike of electrical discharge that travels along the membrane of the cell. Electrophysiology normally involves placing electrodes into preparations of biological tissue.

In the context of drug screening the usefulness of a compound is often evaluated on the basis of its effect on the electrical activity of the cells. Measuring the electrical activity of a cell can be achieved by using a patch-clamp technique, where a small portion (patch) of a cell membrane is attached (clamp), by suction or by means of an electric field, to a tip portion of a glass micropipette with a microelectrode probe; the glass tip forms a high resistance seal (in the order of GΩ) with the cell membrane.

A classical patch-clamp technique is time consuming and requires special skills for the preparation and manipulation of a micropipette. Therefore it is not suitable for screening a large quantity of candidate compounds for a drug at high speed.

A variant of classical patch-clamp is planar patch-clamp; herein, instead of positioning a pipette on an adherent cell, a cell suspension is pipetted on an electronic chip containing a micro-aperture. Then a cell is positioned in the aperture, with the result that a seal with an impedance of the order of giga-ohms is formed. This allows recording variations in the extracellular potentials caused by transmembrane currents.

W02006031612 (Molecular Devices Inc.) is a good example of planar patch-clamp technology. It discloses a system for analysis of membranous samples having ion channels including at least one membranous sample, a multicompartment structure including an extracellular chamber, an opposing intracellular chamber and a partition separating the extracellular and intracellular chambers, the partition having a plurality of apertures fluidly and electrically coupling the extracellular and intracellular chambers, wherein at least one of the apertures is sealed by the at least one membranous sample, and another of the apertures is unsealed, an electric source being configured to apply a current between the extracellular and intracellular chambers, wherein a portion of the current travels through the unsealed aperture, and a current sensor being configured to measure the current between the extracellular and intracellular chambers.

In another variant, Multielectrode arrays (MEAs) seek to increase the throughput of electrophysiological recordings by supporting the cell culture on arrays of micrometer-sized electrodes (typically made of platinum, Indium Tin Oxide or gold) embedded in glass substrates. Each microelectrode can record action potentials extracellulary, i.e., without cell membrane disruption, as long as the distance between the microelectrode and the axon-hillock is small (theory predicts a limit at several tens of micrometers). Microelectrode numbers in the order of 100 are possible, so that multiple neurons can be recorded from a single culture. When drugs are applied to a neuronal culture on a MEA, the resulting changes in activity can be recorded as variations in the spiking rate at multiple microelectrodes simultaneously.

It is widely accepted that measurements of extracellular potentials using MEAs require the size of each recording electrode to be comparable to the size of the cell. If an electrode is not substantially covered by a cell, it is not considered feasible to obtain a recordable signal from that electrode.

It is difficult to position and to keep single cells at a defined location close to the recording electrode. The use of microwell and microelectrode arrays partially solve this problem, because the microwells physically constrain the movement of the cells to the proximity of the recording site, keeping them in place during recording and stimulation in a defined extracellular volume. Some neurons happen to fall within tens of microns of a recording site and produce recordable extracellular spikes when active.

But producing such microwell and microelectrode arrays involves photolithography technology and clean-room manufacturing, which is expensive and labour-intensive and, hence, seriously limits their scope for high throughput screening.

Besides, high throughput approaches using planar patch-clamp techniques is viable for cells with smooth surfaces sealing against arrays of micro-apertures by virtue of electric field forces, suction or other means. However, the irregular shape and surface structure of adherent cells, such as nerve cells and myocytes, makes difficult the formation of a high-impedance seal on such micro-apertures.

As an alternative to photolithography, a soft-lithographic microfluidic structure has been described (*"*Extracellular recordings of field potentials from single cardiomyocytes". Klauke et al., Biophysical Journal, Volume 91, October 2006) for the exploration of heart electrophysiology, which enables extracellular voltages and currents to be measured with extracellular electrodes which are not in contact with the cell. An array of microchannels was formed by micro-moulding in a polymer film, and an insulating polymer partition was lithographically formed to define two microfluidic pools to enable the two ends of the myocyte to be physiologically manipulated independently of each other. Specifically, arrays of parallel microchannels (40 µm wide, 12-15 µm high) with integrated planar microelectrodes (40 µm * 20 µm) were fabricated on microscope coverslips; the microchannels in the arrays were aligned along their longitudinal axis with a 20 or 40 µm wide gap between adjacent channels.

However, this approach still relies on expensive microsystems technology because MEA-type substrate-embedded microelectrodes need be microfabricated.

Claverol et al. ("Multisite recording of extracellular potentials produced by microchannel-confined neurons in-vitro". IEEE transactions on biomedical engineering, volume 54, February 2007) have described the fabrication of hybrid elastomer-glass devices with a polymer-on-microelectrode array for recording extracellular potentials from confined neurons, as well as a device and method of spatially localized multisite recordings from individual microchannel-guided neurites (axons or dendrites) extending from microwell-confined somas. These microwell and microchannel multisite devices are made from polymeric microstructures suitable for cell guiding using soft-lithography technology.

Yet, in that work, MEAs are fabricated in clean-room facilities by photolithographically defining a set of parallel ITO microelectrodes on a glass substrate, which is expensive, and the operation of MEAs is based on getting cells close to the lithographically fabricated non-movable microelectrodes, whereby the probability of a microelectrode recording signals is less than 1 because the distance between the axon-hillock and the recording site cannot be accurate enough when using conventional random positioning of cells. Therefore MEAs are still not economically feasible for large-scale screening of candidate drugs, and they can no be treated as consumables due to their cost.

### SUMMARY OF THE INVENTION

It is an object of the present invention to provide a system for recording electrical activity produced by electrogenic cells in-vitro such that high throughput screening of drugs for specific effects on electrical activity is economically feasible.

With the invention, adherent cells, for example neurons, are cultured on substrates at the bottom of wells with at least one duct connected to the well, at the level of the cell culture substrate.

Upon plating and as part of normal growth in-vitro, cells flatten and extend laterally, sprouting neurites in the case of neurons and multiplying in the case of cell lines. As these processes take place, parts of the cells eventually enter the duct or partially occlude its entrance bur normally leaving a narrow electrolyte gap inside the duct. The flow of transmembrane current in the cells of the duct and this gap results in changes of the electrical potential in the well to which the duct is connected with respect to the opposite end of the duct, and this potential can be recorded by a couple of electrodes.

According to one aspect of the invention, the width of one well of the device is at least ten times the width of the duct, preferably at least twenty times and more preferably at least fifty times the width of the duct, and the width of one electrode is at least ten times the width of the duct. By width it is meant the distance from side to side as seen in the drawings (including possible diagonal distances), but the term is not limited to horizontal dimensions. If the cross-section of the well is not circular or square or when it is variable (for example, when the well is a cone), the well's width means the shortest cross-section distance (the largest being the length).

The duct's width must be large enough to allow cell outgrowth along it but must remain sufficiently narrow to produce recordable electrical signals. The well's width, instead, need not be of the order of the duct's width but can be much larger, because it is not necessary to guide any particular cell towards the duct's opening. The electrode's width can then be of the same order as the well's. It should be noted that manufacturing normal-sized wells and electrodes (of the order of tenths of millimetres to several millimetres) is cheaper than micro-manufacturing them (below 50 micrometers).

Preferably, the axial direction of the duct is substantially perpendicular to the axial direction of the well. Since the axial direction of the well is, in operation, substantially vertical (the well is normally open at its top region), it follows that the axial direction of the duct is substantially horizontal and that the duct opening is substantially vertical. As the cell does not lie then on the duct opening, it is not likely for the cell to make a seal but to adhere to the vicinity of the duct opening.

The length of the duct is preferably of at least 50 µm (micrometers), and more preferably of at least 100 µm, so that it may house a suitable part of the cell and produce a recordable signal.

Advantageously, at least one electrode is reusable, so that it can be placed in and out of said well.

Preferably, the polymeric structure comprises two such wells connected by said duct, and two electrodes such that one of said electrodes can be placed in one well and the electrode can be placed in the other well; for example, the electrodes can be lowered in and lifted out of the wells. Alternatively, one of said electrodes is located in one well and the other electrode is located in the other well, for example integral with them.

In an embodiment, the device comprises a plurality of such wells, ducts and electrodes, so that at least some electrodes may be arranged in a reusable set which can be placed in and out of a corresponding set or array of wells.

In an embodiment, the device comprises a transparent substrate for the polymeric structure, so that at least some electrodes may be fixed to the substrate, for example embedded in or on it.

In an embodiment, the width of at least one well is at least 0.1 mm. In an embodiment, the width of at least one electrode is at least 0.1 mm. In an embodiment, the height of the ducts is in the range 1-30 µm. Thus the duct's width and height are at the micro-level (less than 50 µm) but they can still be made at low cost because they can be moulded in the polymeric structure, and the well's and electrode's width are at the macro-level (more than 100 µm) so that they do not require clean-room micro-fabrication. The polymeric structures and the electrodes can even be made reusable.

In an embodiment, the device comprises a cell culture dish; for example the polymeric structure can be placed in a standard plastic dish.

According to another aspect of the invention, an apparatus for recording cellular electrical activity in-vitro comprises a device according to the invention.

In an embodiment, said apparatus comprises an electronic system for processing the signals recorded by the electrodes, for example a multichannel system for data acquisition and data analysis that may allow for parallel high throughput screening of compounds for pre-specified effects on neuronal activity.

Preferably, the electronic system comprises an electronic subsystem for electrical stimulation of activity in the cell culture. This stimulation can be delivered through the electrodes.

Advantageously, the electronic system comprises a computing system.

In an embodiment, the apparatus comprises a computer-controlled pump for releasing compounds in the wells.

In an embodiment, the apparatus comprises a microscope for performing optical assays. That's why the substrate, when present, is preferably transparent.

According to another aspect of the invention, a method of recording cellular electrical activity in-vitro comprises using a device or an apparatus according to the invention.

Preferably, said method comprises the step of placing a recording electrode in a first well and a reference electrode in a second well connected to the first well, both wells containing cultured cells, and the step of measuring the electrical potential between said two electrodes.

According to another aspect of the invention, a method of manufacturing a device according to the invention comprises using replica mould technology, which is a simple and cheap technology.

In an embodiment, the method comprises the step of setting (for example, by pouring or spinning) the polymer to the desired thickness on a master comprising microstrips of a photoresistant material but devoid of columns. The microstrips produce the ducts but the wells are produced in a further step, for example by punching.

The devices, apparatuses and methods defined by the present invention are an alternative to photolithographically fabricated multielectrode arrays, that offer similar but lower-cost multichannel recordings. One application of this technology is massive drug screening.

The present invention can be used to evaluate candidate antagonist and agonist compounds by comparison of the temporal profile of the recorded signals before and after application of the drug.

The invention can further be used to record transmembrane currents associated with a specific channel using non-electrogenic cells such us HeLa, COS and other cell lines transfected with the gene coding of said transmembrane channel. The cells conform to the elongated shape of the micro-duct which confines the extracellular currents flowing through the transfected channel and produces recordable signals.

### BRIEF DESCRIPTION OF THE DRAWINGS

Some embodiments of the present invention will be described in the following, only by way of non-limiting examples, with reference to the appended drawings, in which:
figure 1 is a schematic vertical cross-sectional view of a conventional planar patch-clamp device known in the art;
figure 2 is a schematic vertical cross-sectional view of an embodiment of a device according to the invention;
figure 3 is a schematic vertical cross-sectional view of another embodiment;
figure 4 is a schematic vertical cross-sectional view of another embodiment;
figure 5 is a schematic horizontal cross-sectional view of the embodiment of figure 4;
figure 6 is a device comprising an array of devices as the one in figure 5; and
figure 7 is a schematic horizontal cross-sectional view of another embodiment of a device according to the invention.

The drawings are purely schematic representations and are not intended to reflect actual proportions.

### DESCRIPTION OF PARTICULAR EMBODIMENTS

Throughout this description many references to neurons will be made. Unless otherwise stated these references are to be understood as encompassing other suitable electrogenic cells, such as myocytes.

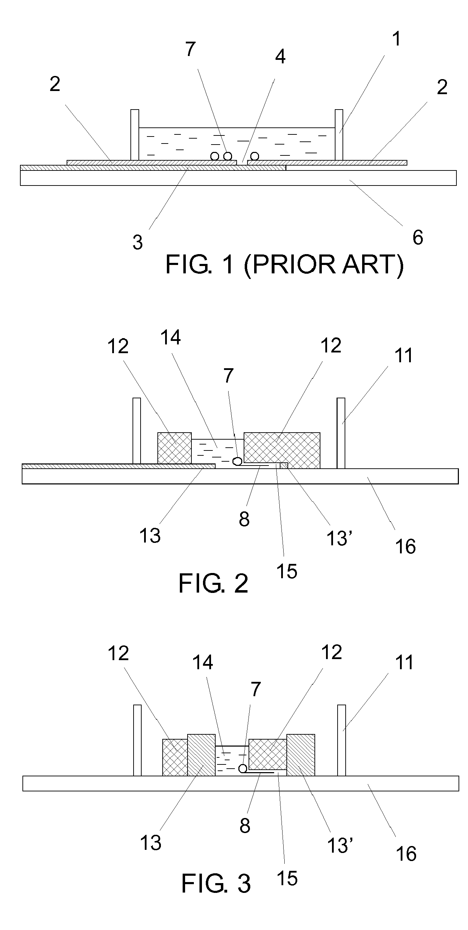

Figure 1 shows a device for recording electrical activity of neurons that is already known in the art. It may be part of a planar MEA and comprises a cell culture dish 1 the bottom of which is an electrically insulating layer 2. There is a conducting layer (or electrode) 3 under the insulating layer and there is an access to the conducting layer inside the dish, through an opening 4 in the insulating layer. The whole is supported by a glass substrate 6. The diameter (or width) of the opening 4 is of the same order of magnitude as the diameter of the soma of a neuron (although the thickness of layer 2 can be much shorter), and one or more neurons 7 are expected to adhere to the layer 2 in the vicinity of the opening 4 and to produce electrical activity recordable by the electrode 3 (with a reference electrode not shown).

Figure 2 shows an embodiment of a device according to the present invention. It comprises a dish 11 to the bottom of which a polymeric structure 12 is attached. Polymers suitable for the polymeric structure 12 are, for example, PDMS (polydimethylsiloxane), which is an elastomer, and polystyrene.

The polymeric structure 12 comprises a well 14 from a side of which a microchannel or duct (a micro-duct really) 15 opens away; the duct 15 is close-ended and opens from the bottom region of the well 14. A couple of electrodes 13 and 13' can record the difference of potential between both ends of a neuron 7 adhered to the vicinity of the opening of or entrance to the duct 15, one end (soma) being in the well 14 and the other (neurite) end being in the duct 15. The active electrode 13 is located in the well 14, not very near the duct's opening, and the reference electrode 13' is located at the closed-end of duct 15, so that electrode 13 can be much larger than electrode 13'. Electrodes 13 and 13' are connected to a signal processing system (not shown) comprising an amplifier. Metals suitable for the electrode are, for example, silver and silver chloride. The whole is supported by a glass substrate 16.

The well 14 houses a cell culture and the duct 15 is narrower than a neuron soma. In these circumstances it is very likely that cell growth in-vitro will result in a neuron 7 extending at least one neurite 8 into the duct 15, but the neuron soma will remain in the well 14. The duct establishes in this way a loose-patch relationship with the neuron; the impedance of the duct-neurite gap is of the order or mega-ohms, contrasting with a typical patch-clamp seal which is of the order of giga-ohms. Then signals produced by the neurite can be detected by the electrode 13 (with respect to the reference electrode 13') and processed by the signal processing system. Other cellular types besides neurons are also compatible with this device; in some cases portions of the cell bodies might grow inside the duct almost sealing its opening.

The diameter of the soma of a neuron is in the range 10-50 µm and, for the case of neuronal tests, the duct 15 may be 50-2000 µm long, 10-40 µm wide and 5-10 µm high, the well's width 14 may be in the range 0.1-20 mm and the electrode's width may be similar to the well's. As to the well's height, the well has to be just high enough to house the cell culture at a level above the duct 15; the well's height is then usually in the range 0.1-10 mm.

The width of the well 14 (and that of electrode 13 too) is at least ten times the width of the duct 15, and preferably 50-fold, so that the duct's size is at the micro-level (as suits the neurite's size) while the well's size is at the macro-level, which is easier to manufacture.

Throughout this specification the term "width" is intended to mean "extent from side to side", and when used it is preferred to the term "diameter" because it may refer to a non-circular cross-section; with reference to the figures as represented the width of an element has the usual meaning of horizontal side to side distance. Thus the well's width is a horizontal distance on the plane of the drawings but the duct's width is a depth as viewed from the plane of the drawings.

Yet it should be noted that although the width of something usually relates to a horizontal dimension, in this specification this may not always be the case; for example, if the dish 11 is tilted the width of the well 14 or of the duct 15 may no longer be a horizontal dimension. Obviously heights are represented in the drawings as vertical distances.

Figure 3 represents an embodiment similar to the embodiment of figure 2 but with electrodes 13 and 13' configured differently. In this case both electrodes are relatively large and connect to the signal processing system (not shown) through the top region of the device, while in the embodiment of figure 2 the connections are through the bottom region of the device.

In a variant of the device shown in figure 3, the active electrode 13 can provide one wall of the well 14, just like the reference electrode 13' provides the closed-end of the duct 15.

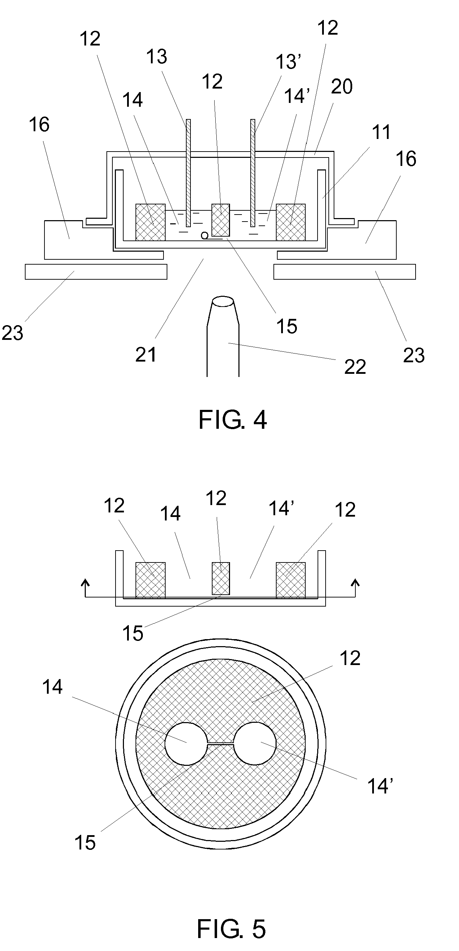

Figure 4 shows another embodiment of the present invention. It comprises a cell culture dish 11 which contains a polymeric structure 12. The polymeric structure 12 comprises two wells 14 and 14' and a duct 15 connecting the bottom region of the two wells. The width of the wells 14,14' is at least ten times the width of the duct 15, and preferably 50-fold, so that the duct's size is at the micro-level while the well's size is at the macro-level.

The dish 11 may be provided with a removable cap 20 and may be supported by a plate 16. Two removable electrodes 13,13' can be lowered into the two wells 14,14', respectively, and lifted out of them. Electrodes 13,13' may operate with a reference electrode (not shown) or electrode 13' may be the reference electrode for electrode 13, and vice versa. The electrodes are connected to a signal processing system (not shown), for example through the plate 16.

The dimensions of wells, electrodes and duct are like in the previous embodiment (as seen in figure 4, the width of the electrodes 13,13' is a depth as viewed from the plane of the drawing, just like the width of the duct 15).

As the neurons spike, the extracellular current associated with the transient membrane potential changes is confined to the duct and a measurable potential appears between the wells connected by the duct.

One way of operating this device is then culturing a population of neurons in the wells 14,14', having one or more neurons extend a neurite into the duct 15, measuring spontaneous or electrically stimulated neuronal activity as changes in the potential difference between the two wells 14,14' by means of the two electrodes 13,13', and recording and processing said potential measurement. So it is not necessary to place an electrode very near a particular neuron neither to have the electrode's or the well's size at the micro-level (of the order of the neuron's size).

The plate 16 may have an opening (or a transparent portion) 21 through which optical assays on cells growing in the wells can be performed with a microscope 22. The plate 16 can be laid on a microscope stage 23.

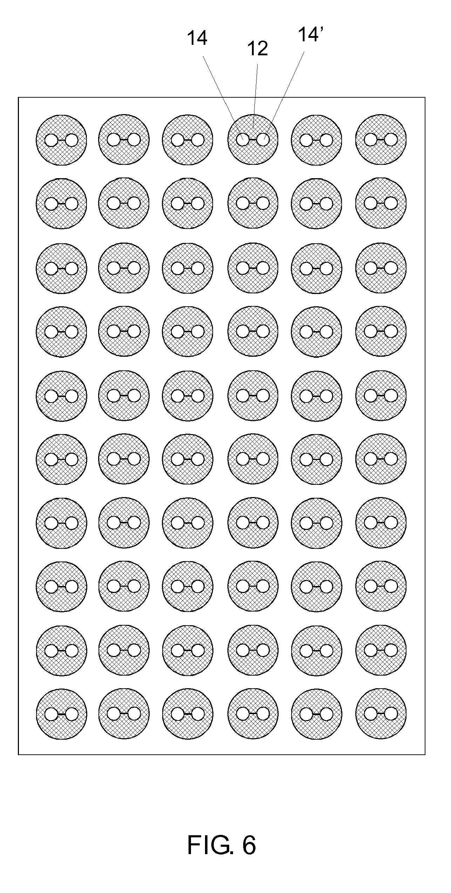

Figure 5 shows a cross-section of the bottom region of the polymeric structure 12 which includes the wells 14,14' and the duct 15, and figure 6 represents a new embodiment comprising a plurality of groups of two wells and a duct as the one shown in figure 4. In figure 6 the two wells plus a duct groups form an ordered array. With this embodiment a high throughput screening of drugs is feasible.

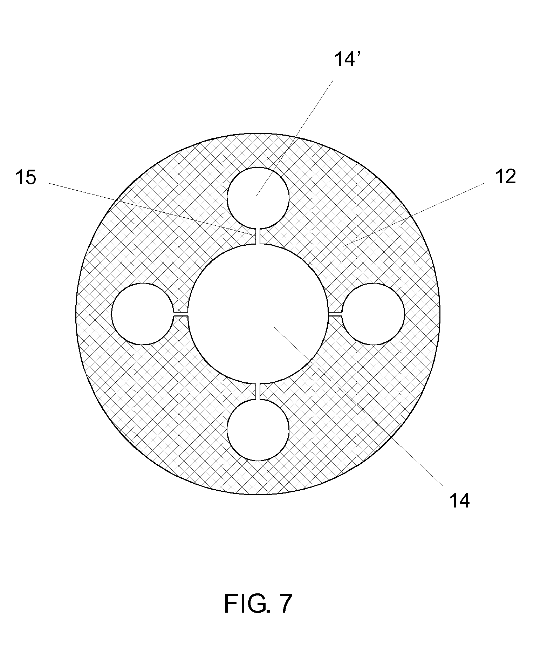

Figure 7 shows yet another embodiment of the device, in which one central well 14 pairs with several smaller wells 14' configured concentrically around the central well 14. As before, each pair of wells 14 and 14' is connected by a much narrower duct 15, and the whole is comprised in a polymeric structure 13.

A computer controlled pump can release compounds in the wells or chambers. Suitable software can control the pump or pumps and can obtain spike-rate versus compound concentration curves (prototypical drug screening assay).

The described devices do not require special cell culture protocols or special cell culture surfaces and are compatible with virtually any cell culture plastic-ware and glass-ware.

A prototype of a polymeric structure as described above can be readily produced by means of a laser-write system. But for mass production of said polymeric structures (especially arrays) other methods are preferred, such as the following one.

The polymer is poured or spun to the desired thickness on a master containing microstrips of a photoresist, such as SU-8 (polyester resin) or similar, but devoid of columns. It is cured (either by heat, UV or other means) and peeled off to yield slabs of polymer populated by ducts at the micro-level. The masters produced in this step can be produced with a single-step photolithographic technique or by means of laser-write lithography.

The slabs are then overlaid on a soft substance (e.g, a PDMS block) and wells are punched through employing a customised array of biopsy-punch cylinders of the desired radius (typically 1-10 mm wide but other diameters are possible). Alignment of the biopsy-punch array to the micro-duct engraved slab before punching is performed with the aid of a x-y-z stage and a rotation stage.

The resulting perforated slab is peeled off the punching bed, aligned to the cell culture plastic-ware or glass-ware of choice, and attached. Conformant attachment of PDMS to popular culture plastics, such as polystyrene or borosilicate glass is sufficient for long-term recordings of neuronal signals. Should detachment due to mechanical stress or harsh culture procedures be a concern, oxygen plasma treatment could be performed on the polymer structures for irreversible attachment to plastics or glass.

Although only particular embodiments of the invention have been shown and described in the present specification, the skilled man will be able to introduce modifications and substitute any technical features thereof with others that are technically equivalent, depending on the particular requirements of each case, without departing from the scope of protection defined by the appended claims.

For example, multiple geometries of the wells and the ducts are possible, including groups of two, three or more wells with interconnecting ducts. They have in common with the described embodiments some micrometer sized cavities (ducts) containing parts of the cells, and wells and electrodes at the macro-level.

Or, instead of lowering the electrodes in the wells they can be inserted through the bottom substrate to access the liquid in the wells.

## Claims

1. Device for recording electrical activity produced by cells in-vitro, comprising a polymeric structure (12) which comprises a well (14) for housing and culturing cells, a duct (15) connected to the bottom region of said well, and two electrodes (13,13') for recording a potential in said well, **characterized in that** the width of said well is at least ten times the width of said duct and the width of at least one of said electrodes (13) is at least ten times the width of said duct.

2. Device according to claim 1, wherein the width of said well is at least twenty times the width of said duct.

3. Device according to claim 1, wherein the width of said well is at least fifty times the width of said duct.

4. Device according to any of the preceding claims, wherein the axial direction of the duct (15) is substantially perpendicular to the axial direction of the well (14).

5. Device according to any of the preceding claims, wherein the length of the duct (15) is of at least 50 micrometers.

6. Device according to any of the preceding claims, wherein the length of the duct (15) is of at least 100 micrometers.

7. Device according to any of the preceding claims, wherein at least one of said electrodes (13) is reusable, so that it can be placed in and out of said well.

8. Device according to any of the preceding claims, wherein the polymeric structure comprises two such wells (14,14') connected by said duct (15).

9. Device according to claim 8, wherein one of said electrodes (13) can be placed in one well (14) and another of said electrodes (13') can be placed in the other well (14').

10. Device according to claim 9, wherein one of said electrodes (13') is located in one well (14) and another of said electrodes (13') is located in the other well (14').

11. Device according to any of the preceding claims, which comprises a plurality of such wells, ducts and electrodes.

12. Device according to claim 11, wherein at least some electrodes are arranged in a reusable set which can be placed in and out of a corresponding set of wells.

13. Device according to any of the preceding claims, which comprises a transparent substrate (16) for the polymeric structure (12).

14. Device according to claim 13, wherein at least some electrodes are fixed to the substrate.

15. Device according to any of the preceding claims, wherein the width of at least one well is at least 0.1 mm.

16. Device according to any of the preceding claims, wherein the width of at least one electrode is at least 0.1 mm.

17. Device according to any of the preceding claims, wherein the height of the ducts is in the range 1-30 micrometers.

18. Device according to any of the preceding claims, which comprises a cell culture dish (11).

19. Apparatus for recording cellular electrical activity in-vitro, **characterized in that** it comprises a device according to any of the preceding claims.

20. Apparatus according to claim 19, which comprises an electronic system for processing the signals recorded by the electrodes.

21. Apparatus according to claim 20, wherein the electronic system comprises an electronic subsystem for electrical stimulation of activity in the cell culture.

22. Apparatus according to claim 20 or 21, wherein the electronic system comprises a computing system.

23. Apparatus according to claim 22, which comprises a computer-controlled pump for releasing compounds in the wells.

24. Apparatus according to any of claims 19 to 23, which comprises a microscope (22, 23) for performing optical assays.

25. Method of recording cellular electrical activity in-vitro, **characterized in that** it comprises using a device or an apparatus according to any of the preceding claims.

26. Method according to claim 25, which comprises the step of placing a recording electrode (13) in a first well (14) and a reference electrode (13') in a second well (14') connected to the first well (14), both wells containing cultured cells, and the step of measuring the electrical potential between said two electrodes.

27. Method of manufacturing a device according to any of claims 1 to 18 that comprises using replica mould technology.

28. Method according to claim 27, which comprises setting the polymer to the desired thickness on a master devoid of columns but comprising microstrips of a photoresistant material.
